(19) Europäisches Patentamt
European Patent Office
Office européen des brevets

(11) Publication number: **0 493 778 B1**

# (12) EUROPEAN PATENT SPECIFICATION

(45) Date of publication of patent specification: **17.05.95**

(51) Int. Cl.[6]: **C07D 301/12**, C07D 303/00, C07D 303/04

(21) Application number: **91122123.2**

(22) Date of filing: **23.12.91**

(54) **Process for the catalytic mono-epoxidation of diolefins.**

(30) Priority: **24.12.90 IT 2254090**

(43) Date of publication of application:
**08.07.92 Bulletin 92/28**

(45) Publication of the grant of the patent:
**17.05.95 Bulletin 95/20**

(84) Designated Contracting States:
**BE CH DE FR GB IT LI NL**

(56) References cited:
**EP-A- 0 109 273**
**EP-A- 0 190 609**

**JOURNAL OF ORGANIC CHEMISTRY, vol. 53, no. 7, 1st April 1988, pages 1553-1557, Easton, US; C. VENTURELLO et al.: "Ouaternary ammonium tetrakis(diperoxotungsto)phosphates(3-) as a new class of catalysts for efficient alkene epoxidation with hydrogen peroxide"**

(73) Proprietor: **ENICHEM S.p.A.**
**Piazza Repubblica, 16**
**I-20124 Milano (IT)**

(72) Inventor: **Ricci, Marco, Dr.**
**19, Via Fra Dolcino**
**I-28100 Novara (IT)**
Inventor: **Ungarelli, Raffaele**
**1, Via Tanaro**
**I-28069 Trecate (Novara) (IT)**
Inventor: **Venturello, Carlo, Dr.**
**135, Corso XXIII Marzo**
**I-28100 Novara (IT)**

(74) Representative: **Barz, Peter, Dr. et al**
**Patentanwälte Dipl.-Ing. G. Dannenberg**
**Dr. P. Weinhold, Dr. D. Gudel**
**Dipl.-Ing. S. Schubert, Dr. P. Barz**
**Siegfriedstrasse 8**
**D-80803 München (DE)**

EP 0 493 778 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**CHEMICAL ABSTRACTS vol. 103, no. 17, 28th October 1985, page 703, abstract no. 141761f, Columbus, Ohio, US; Y. MATOBA et al.: "Hydrogen peroxide-oxidation of olefins by 12-molybdatophosphoric acid in the presence of quaternary ammonium salts", & KENKYU HOKOKU - ASAHI GARASU KOGYO GIJUTSU SHOREIKAI 1984, 44, 77-81**

**JOURNAL OF ORGANIC CHEMISTRY, vol. 48, no. 21, 21st october 1983, pages 3831-3833, Easton, US; C. VENTURELLO et al.: "A new, effective catalytic system for epoxidation of olefins by hydrogen peroxide under phase-transfer conditions"**

**D. Swern (Editor), Organic Leroxides, vol. II, Wiley-Interscience, New York, p. 454**

## Description

The present invention relates to a process for the mono-epoxidation of symmetrical diolefins by means of a catalytic oxidation reaction in a liquid phase.

In particular, the invention relates to a process for the preparation of mono-epoxides by means of oxidation of symmetrical diolefins in a liquid phase with hydrogen peroxide in the presence of a peroxidic catalyst based on tungsten.

More specifically, the present invention relates to the preparation of mono-epoxides, starting from unconjugated symmetrical diolefins and hydrogen peroxide in the presence of the above catalyst and under phase transfer conditions.

The compounds obtained, i.e., mono-epoxides of symmetrical diolefins, are chemical products of considerable industrial importance.

In fact, they represent products which can be applied in a wide range of fields well-known to those skilled in the art.

Besides being useful intermediates for organic syntheses, the mono-epoxides of some diolefins, for example linear alpha,omega-diolefins, are particularly used as grafting agents for polymers such as polyethylene terephthalates.

The preparation of dienic mono-epoxides poses considerable problems, especially with respect to an acceptable selectivity, with the result that it is often carried out by means of fermentation (with consequent problems of low productivity, applicability limited to certain structures etc.) or by methods of little practical interest such as the reaction of Grignard reagents with suitable halogen derivatives (H.C. Brown, G.J. Lynch, J. Org. Chem. 1981, 46, 930; A. De Camp Schuda, P.H. Mazzocchi, G. Fritz, T. Morgan, Synthesis 1986, (309)), or by means of the decomposition of aliphatic diazonium salts (W. Kirnse, U. Jansen, Chem. Ber., 1985, 118, 2607).

Even the selective epoxidation of diolefins with percarboxylic acids, which in certain cases represents the only practical and general method for the preparation of mono-epoxides, is not without drawbacks. This method, in fact, affords mixtures of mono- and di-epoxide: the higher the conversion of the substrate (or, in other words, the higher the ratio oxidant/diolefin), the higher will be the content of diepoxide in these mixtures, although a certain amount of diepoxide is also formed when equimolar amounts of reagents or even an excess of diolefin are used. The recovery of the mono-epoxide from these mixtures and its purification, consequently, require time-consuming, accurate distillation operations which considerably reduce the productivity of these processes. Moreover, the use of peracids causes other disadvantages with respect to the slowness of the reactions, the safety of the process, corrosion of the equipment, the cost of reagents and the recovery and disposal of considerable quantities of carboxylic acids coming from the reduction of the peracid.

A method for the transformation of olefins into the corresponding epoxides by reaction with hydrogen peroxide in a biphasic water/organic system and in the presence of a peroxidic catalyst based on tungsten has also been described (EP-A-109 273). This process, however, is basically directed towards the epoxidation of mono-olefins and, in any case, does not give any indication as to the selectivity of the mono-epoxidation with dienic substrates.

On the other hand, on the basis of experiments it has been established that the selective mono-epoxidation of dienic substrates is influenced by:

(a) the nature of the two double bonds;
(b) the epoxidizing system used;
(c) the hydrophilic degree of the epoxides obtained.

In J. Org. Chem, 53, 1553 - 1557 (1988) there is disclosed, i.a., the catalytic epoxidation of an asymmetrical diolefin, i.e., 4-vinyl cyclohexene, by means of a quaternary ammonium tetrakis-(diperoxotungsto)phosphate(3-) in conjunction with hydrogen peroxide as the primary oxidant in an aqueous/organic biphase system. Said epoxidation affords mainly the monoepoxide (reaction at the ring double bond).

EP-A-190609 describes the monoepoxidation of, i.a., symmetrical diolefins by means of hydrogen peroxide in the presence of a titanium-silicalite as catalyst in a heterogeneous system.

Object of the present invention is the provision of a selective catalytic process for the mono-epoxidation of unconjugated symmetrical diolefins, which is both easy to carry out and economical.

This and other objectives, which the following description will make evident are achieved, according to the present invention, by a catalytic process for the mono-epoxidation of diolefins, wherein a symmetrical diolefin of general formula (I):

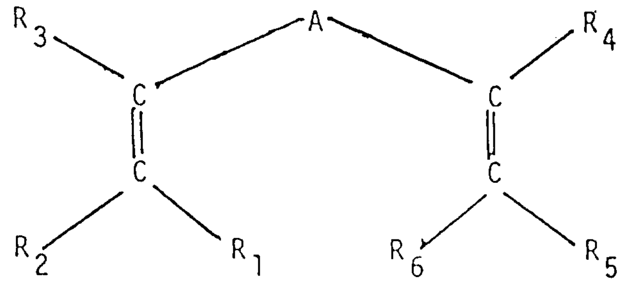

(I)

wherein $R_1$, $R_2$, $R_3$, $R_4$, $R_5$ and $R_6$,

represent hydrogen, methyl or ethyl and the groups $R_1$ and $R_6$ together may also form a group $-(CH_2)_n-$ wherein n ranges from 1 to 5; and A represents an optionally substituted aliphatic hydrocarbon chain containing from 1 to 6 carbon atoms or an optionally substituted aromatic or hetero-aromatic group (e.g. phenylene) containing up to 6 carbon atoms, and wherein the diolefin may also comprise one or more groups selected from ether, carbonate, halogen, nitro, alkoxy, carbonyl, carboxy and ester groups , provided that the total number of carbon atoms of A, $R_1$, $R_2$, $R_3$, $R_4$, $R_5$ and $R_6$ without said optional substituents is not higher than six; is reacted with hydrogen peroxide in a liquid biphasic system composed of:

(a) an organic phase comprising (essentially containing) the diolefin and one or more catalysts of general formula (II):

$Q_3XW_4O_{24}$ (II)

wherein Q represents a quaternary ammonium or phosphonium cation containing up to 70 carbon atoms, preferably from 25 to 40 carbon atoms, and X represents P or As; and, optionally, a solvent; and

(b) an aqueous phase comprising (essentially containing) hydrogen peroxide.

Alternatively, the catalyst can be supported in the conventional way resulting, in this case, in a third solid phase (c). As a further alternative, the catalyst can be prepared "in situ".

In this way, mono-epoxides of diolefins of formula (I) may be obtained with high selectivity.

Preferably, the amount of diepoxide is kept low independent, within certain limits, of the conversion of the substrate or, in other words, from the ratio oxidant/diolefin. This surprising characteristic allows to simplify the recovery and purification of the mono-epoxide and to eliminate the necessity of a highly accurate dosing of the oxidant.

The reaction, as specified above, is carried out in a biphasic water-organic system, usually under vigorous stirring, and in the presence of a catalyst.

In particular, the diolefins of formula (I) are diolefins whose double bonds:

(a) are not in a "conjugated" position and

(b) are equivalent, the term "equivalent" referring to their equal reactivity with respect to the oxidizing system, as intended in the present invention.

As already specified, the total number of carbon atoms present in the diolefin skeleton ranges form 5 to 10. However, the presence of hydrophilic groups or functions in the molecule may result in a higher total number of carbon atoms.

Included in the above definition of diolefins of formula (I) are diolefins having inert substituent groups, such as ether, carbonate, halogen (e.g. F, Cl, Br), nitro, alkoxy (e.g. $C_1$-$C_4$ alkoxy), carbonyl, carboxy and ester groups.

Diolefins suitable for epoxidation in accordance with the present invention are, for example, 1,5-hexadiene, 1,7-octadiene, 1,9-decadiene, diallylether, diallylcarbonate, the three divinylbenzene isomers (o-, m- and p-), 1,4-cyclohexadiene, 1,5-cyclo-octadiene, etc. Preferred diolefins are those wherein $R_1$, $R_2$, $R_5$ and $R_6$ represent hydrogen or wherein $R_2$ and $R_5$ are hydrogen and $R_1$ and $R_6$ together represent a $-(CH_2)_n-$ group with n = 1 to 3.

According to the present invention, the diolefins of formula (I) are selectively mono-epoxidized in the above way, using the previously defined tungsten-based catalysts (II).

Said catalysts belong to a group of compounds already known and described in the literature together with their preparation (e.g. in EP-A-109273 and 232742). Owing to their low price, it is preferable to use catalysts in which Q represents the dimethyl [dioctadecyl (75%) + dihexadecyl (25%)] ammonium ion.

As specified above, the epoxidation reaction is most suitably carried out in a biphasic system essentially composed of an aqueous solution of hydrogen peroxide and an organic phase essentially composed of the diolefin and the catalyst dissolved therein. Sometimes, however, it can prove advantageous to dilute the organic phase with a solvent which is substantially immiscible with water: in this case, examples of solvents which can be used are aromatic hydrocarbons such as benzene, toluene, the xylenes, etc., chlorinated hydrocarbons, such as dichloromethane, trichloromethane, chloroethane, chloropropane, dichloroethanes, trichloroethanes, tetrachloroethanes, di- and tri-chloropropanes, tetrachloropropanes, tetrachloroethylene and chlorobenzene; alkyl esters, such as ethyl acetate; or suitable mixtures thereof.

The choice of the kind of organic phase (a) depends on the reactivity of the starting diolefin and the parameters used. If the above inert solvents are used in the organic phase, the concentration of the diolefin therein is not critical for carrying out the process.

Preferred values of the concentration of the diolefin in the organic phase range from 5 to 95% by weight, higher or lower values being acceptable within the limits of their practical use. Finally, the concentration of hydrogen peroxide in the aqueous phase is usually kept within the range of from 0.1 to 70%, preferably from 15 to 70% by weight.

In the case of diolefins whose epoxides are particularly unstable under acidic conditions, the mono-epoxidation reaction may be carried out by adding small quantities of phosphate or alkaline arsenate and tungstate to the aqueous phase and fixing the pH at the desired value of from 2 to 6 by means of acids or mineral bases.

The operating temperature usually ranges from 0 to 120 °C, preferably from 40 to 80 °C, depending on the reactivity of the diolefin, the catalyst, the desired product, etc.

Generally, the process is carried out at atmospheric pressure. However, if diolefins are used which are extremely volatile at the temperature chosen for the reaction, it is possible to operate at higher pressures, normally ranging from 1.013 to 101.3 bar (1 to 100 atmospheres).

The molar ratio hydrogen peroxide/diolefin is usually kept at from 0.5:1 to 1.5:1, depending on the operating conditions.

The catalyst is generally used in a molar ratio, with respect to the hydrogen peroxide, of from 1:10 to 1:2500, preferably from 1:200 to 1:800.

The duration of the reaction depends on the operating conditions (temperature, quantity of catalyst, reactivity of the diolefin, presence of solvent, etc.). Intervals ranging from a few minutes to several hours are generally sufficient for completing the reaction.

A practical method for carrying out the process of the present invention will be described in the following.

The reactants ($H_2O_2$ and the diolefin in the optional solvent) are placed, in predetermined amounts and ratios, in a reactor equipped with stirrer, thermoregulating system and reflux cooler. The catalyst is then added. The heterogeneous mixture is brought, under vigorous stirring, to the temperature of the reaction, for the desired period of time. At the end of the reaction, after de-mixing, cooling etc., the mono-epoxide and the other compounds and reagents may be separated by use of conventional methods (distillation, etc.).

Because of its mild and simple operating conditions, the present process is extremely advantageous, and in particular, allows an effective and rapid mono-epoxidation with high concentrations of diolefin in the solvent or even without a solvent, with the resulting technological and economical advantages.

Moreover, the selectivity of the mono-epoxidation reaction allows to operate also with an excess of hydrogen peroxide without adversely affecting the selectivity.

A more detailed description of the invention is given in the following examples which are provided for illustrative purposes only.
The symbol w/v stands for weight/volume.

EXAMPLE 1

1.71 g of tetra(diperoxotungsto)phosphate of [dioctadecyl (75%) + dihexadecyl (25%)] dimethylammmonium (0.62 mmoles), 22.7 g of 97% 1,7-octadiene (200 mmoles) and 14 ml of water were poured into a 0.5 litre reactor equipped with a mechanical stirrer, thermometer and reflux cooler. The mixture was heated to 70 °C and 35 ml of 17.5% w/v hydrogen peroxide (180 mmoles) were added dropwise over a period of 1.5 hours, with subsequent continuous stirring for a further 20 minutes. The mixture was then cooled, 250 ml of methylene chloride were added and the phases separated. 3 mmoles of residual hydrogen peroxide were determined, by means of iodometric analysis, in the aqueous phase, whereas the organic phase was determined, by gas-chromatography, to contain 69 mmoles of 1,7-octadiene and 95 mmoles of 1,2-epoxy-7-octene, but no 1,2,7,8-di-epoxyoctane. These values correspond to a 98% conversion of the hydrogen peroxide and a selectivity in 1,2-epoxy-7-octene (based on the hydrogen peroxide used) of 54%. The mono-epoxidation selectivity, defined as:

$$\text{"mono"-selectivity} = \frac{\text{mono-epoxide}}{\text{mono-epoxide + di-epoxide}} \times 100$$

was 100%.

EXAMPLE 2 (reaction in solvent)

4.3 g of tetra(diperoxotungsto)phosphate of [dioctadecyl (75%) + dihexadecyl (25%)]dimethyl ammonium (1.56 mmoles), 71.1 g of 97% 1,9-decadiene (500 mmoles), 34 ml of water and 100 ml of 1,2-dichloroethane were poured into a 0.5 litre reactor equipped with mechanical stirrer, thermometer and reflux cooler. The mixture was heated to 50 °C, 29.1 ml of 35% w/v hydrogen peroxide (300 mmoles) were added dropwise over a period of 2 hours, and the stirring was then continued for a further hour. At the end, the mixture was cooled, 250 ml of methylene chloride were added and the phases separated. 7 mmoles of residual hydrogen peroxide were determined, by iodometry, in the aqueous phase, whereas the organic phase was determined, by gas-chromatography, to contain 231 mmoles of 1,9-decadiene, 190 mmoles of 1,2-epoxy-9-decene and 37 mmoles of 1,2,9,10-diepoxydecane. These values correspond to a 98% conversion of the hydrogen peroxide and a selectivity in 1,2-epoxy-9-decene (based on the hydrogen peroxide used) of 65%. The mono-epoxidation selectivity was 84%.

**Claims**

1. Process for the catalytic mono-epoxidation of diolefins, wherein a symmetrical diolefin of general formula (I):

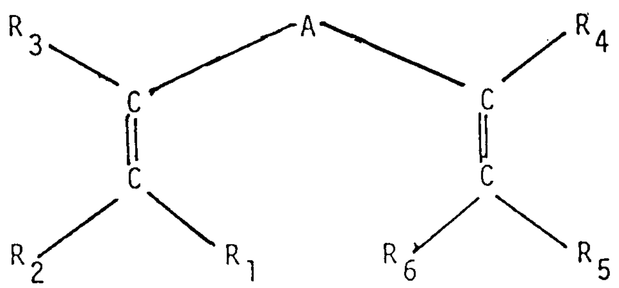

(I)

wherein $R_1$, $R_2$, $R_3$, $R_4$, $R_5$ and $R_6$,

represent hydrogen, methyl or ethyl and the groups $R_1$ and $R_6$ together may also form a group $-(CH_2)_n-$ wherein n ranges from 1 to 5; and A represents an optionally substituted aliphatic hydrocarbon chain containing from 1 to 6 carbon atoms or an optionally substituted aromatic or hetero-aromatic group containing up to 6 carbon atoms, and wherein the diolefin may also comprise one or more groups selected from ether, carbonate, halogen, nitro, alkoxy, carbonyl, carboxy and ester groups , provided that the total number of carbon atoms of A, $R_1$, $R_2$, $R_3$, $R_4$, $R_5$ and $R_6$ without said optional substituents is not higher than six; is reacted with hydrogen peroxide in a liquid biphasic system composed of:

(a) an organic phase comprising the diolefin and one or more catalysts of general formula (II):

$Q_3XW_4O_{24}$ (II)

wherein Q represents a quaternary ammonium or phosphonium cation containing up to 70 carbon atoms, and X represents P or As; and optionally, a solvent; and

(b) an aqueous phase comprising hydrogen peroxide.

2. Process according to claim 1, wherein the diolefin is selected from 1,5-hexadiene, 1,7-octadiene, 1,9-decadiene, diallylether, diallylcarbonate, divinylbenzenes, 1,4-cyclohexadiene and 1,5-cyclooctadiene.

3. Process according to any one of claims 1 and 2, wherein in formula (II) Q represents a quaternary ammonium cation containing hydrocarbyl groups having a total of 25 to 40 carbon atoms and X = P.

4. Process according to any one of claims 1 to 3, wherein a catalyst of formula (II) is used, wherein Q is composed of 75% of dimethyl-(dioctadecyl)-ammonium and 25% of dimethyl-(dihexadecyl)-ammonium and X = P.

5. Process according to any one of claims 1 to 4, wherein the molar ratio of catalyst (II) to hydrogen peroxide ranges from 1:10 to 1:2500, particularly from 1:200 to 1:800.

6. Process according to any one of claims 1 to 5, wherein the molar ratio of hydrogen peroxide to diolefin ranges from 0.5:1 to 1.5:1.

7. Process according to any one of claims 1 to 6, wherein the concentration of hydrogen peroxide in the aqueous phase is from 0.1 to 70% by weight.

8. Process according to any one of claims 1 to 7, wherein the organic phase contains no solvent or at least one inert solvent which is essentially immiscible with the aqueous phase, preferably a solvent selected from aromatic hydrocarbons, chlorinated hydrocarbons, alkyl esters of carboxylic acids and mixtures thereof.

9. Process according to any one of claims 1 to 8, wherein the concentration of the diolefin in the organic phase is from 5 to 95% by weight.

10. Process according to any one of claims 1 to 9, which is carried out at a temperature of from 0 to 120 °C and/or at a pressure of from 1.013 to 101.3 bar (1 to 100 atm).

**Patentansprüche**

1. Verfahren zur katalytischen Monoepoxidation von Diolefinen, bei dem ein symmetrisches Diolefin der allgemeinen Formel (I):

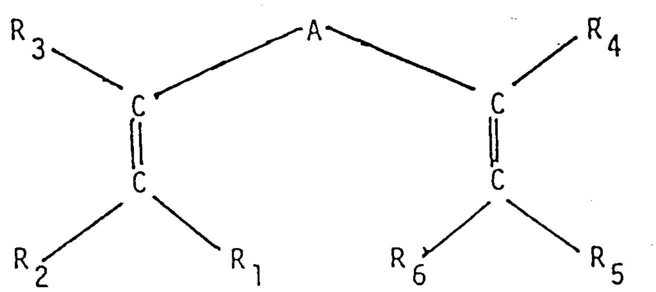

(I)

worin $R_1$, $R_2$, $R_3$, $R_4$, $R_5$ und $R_6$ Wasserstoff, Methyl oder Ethyl darstellen und die Gruppen $R_1$ und $R_6$ zusammen auch eine Gruppe $(-CH_2-)_n$ bilden können, worin n im Bereich von 1 bis 5 liegt; und A eine gegebenenfalls substituierte aliphatische Kohlenwasserstoffkette, die 1 bis 6 Kohlenstoffatome enthält, oder eine gegebenenfalls substituierte aromatische oder heteroaromatische Gruppe, die bis zu 6 Kohlenstoffatome enthält, darstellt und worin das Diolefin auch eine oder mehrere Gruppen umfassen kann, die aus Ether-, Carbonat-, Halogen-, Nitro-, Alkoxy-, Carbonyl-, Carboxy- und Estergruppen ausgewählt sind, mit der Maßgabe, daß die Gesamtzahl der Kohlenstoffatome von A, $R_1$, $R_2$, $R_3$, $R_4$, $R_5$ und $R_6$ ohne diese fakultativen Substituenten nicht höher als 6 ist;
mit Wasserstoffperoxid in einem flüssigen zweiphasigen System umgesetzt wird, das zusammengesetzt ist aus:

(a) einer organischen Phase, die das Diolefin und einen oder mehrere Katalysatoren der allgemeinen Formel (II):

$Q_3XW_4O_{24}$ (II)

worin Q für ein bis zu 70 Kohlenstoffatome enthaltendes quaternäres Ammonium- oder Phosphoniumkation steht und X P oder As bedeutet; und gegebenenfalls ein Lösungsmittel umfaßt;
(b) einer Wasserstoffperoxid umfassenden wäßrigen Phase.

**2.** Verfahren nach Anspruch 1, in welchem das Diolefin aus 1,5-Hexadien, 1,7-Octadien, 1,9-Decadien, Diallylether, Diallylcarbonat, Divinylbenzolen, 1,4-Cyclohexadien und 1,5-Cyclooctadien ausgewählt ist.

**3.** Verfahren nach irgendeinem der Ansprüche 1 und 2, in welchem in Formel (II) Q für ein quaternäres Ammoniumkation steht, das Kohlenwasserstoffgruppen mit insgesamt 25 bis 40 Kohlenstoffatomen enthält, und X = P.

**4.** Verfahren nach irgendeinem der Ansprüche 1 bis 3, in welchem ein Katalysator der Formel (II) verwendet wird, in welchem Q aus 75% Dimethyl-(dioctadecyl)-ammonium und 25% Dimethyl-(dihexadecyl)-ammonium zusammengesetzt ist und X = P.

**5.** Verfahren nach irgendeinem der Ansprüche 1 bis 4, in welchem das Molverhältnis von Katalysator (II) zu Wasserstoffperoxid im Bereich von 1:10 bis 1:2500, insbesondere 1:200 bis 1:800, liegt.

**6.** Verfahren nach irgendeinem der Ansprüche 1 bis 5, in welchem das Molverhältnis von Wasserstoffperoxid zu Diolefin im Bereich von 0,5:1 bis 1,5:1 liegt.

**7.** Verfahren nach irgendeinem der Ansprüche 1 bis 6, in welchem die Konzentration von Wasserstoffperoxid in der wäßrigen Phase 0,1 bis 70 Gewichts-% beträgt.

**8.** Verfahren nach irgendeinem der Ansprüche 1 bis 7, in welchem die organische Phase kein Lösungsmittel oder mindestens ein inertes Lösungsmittel enthält, das mit der wäßrigen Phase im wesentlichen nicht mischbar ist, vorzugsweise ein Lösungsmittel, das aus aromatischen Kohlenwasserstoffen, chlorierten Kohlenwasserstoffen, Alkylestern von Carbonsäuren und Mischungen davon ausgewählt ist.

**9.** Verfahren nach irgendeinem der Ansprüche 1 bis 8, in welchem die Konzentration des Diolefins in der organischen Phase 5 bis 95 Gewicht-% beträgt.

**10.** Verfahren nach irgendeinem der Ansprüche 1 bis 9, das bei einer Temperatur von 0 bis 120 °C und/oder einem Druck von 1,013 bis 101,3 Bar (1 bis 100 atm) durchgeführt wird.

## Revendications

**1.** Procédé de monoépoxydation catalytique de dioléfines, dans lequel on fait réagir une dioléfine symétrique de formule générale (I) :

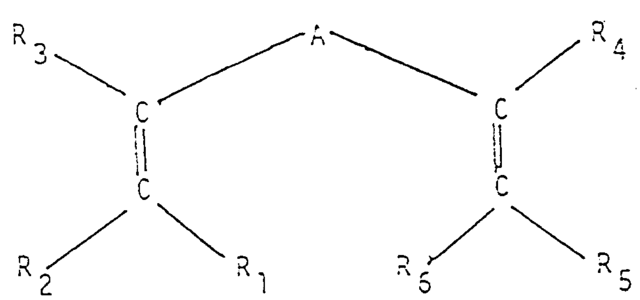

(I)

dans laquelle $R_1$, $R_2$, $R_3$, $R_4$, $R_5$ et $R_6$ représentent des atomes d'hydrogène ou des groupes méthyle ou éthyle, les symboles $R_1$ et $R_6$ pouvant aussi représenter conjointement un groupe $-(CH_2)_n-$ où n vaut de 1 à 5, et A représente une chaîne hydrocarbonée aliphatique éventuellement substituée, comportant de 1 à 6 atomes de carbone, ou un groupe aromatique ou hétéro-aromatique éventuellement substitué, comportant jusqu'à 6 atomes de carbone, cette dioléfine pouvant aussi comporter un ou plusieurs groupes choisis parmi les groupes de types éther, carbonate, halogéno, nitro, alcoxy,

carbonyle, carboxy et ester, pourvu que le nombre total d'atomes de carbone de A, $R_1$, $R_2$, $R_3$, $R_4$, $R_5$ et $R_6$, non compris les éventuels substituants, ne soit pas supérieur à 6, avec du peroxyde d'hydrogène, dans un système liquide biphasique constitué de :

a) une phase organique comprenant la dioléfine et un ou plusieurs catalyseurs de formule générale (II) :

$Q_3XW_4O_{24}$ (II)

dans laquelle Q représente un cation de type ammonium ou phosphonium quaternaire, qui peut comporter jusqu'à 70 atomes de carbone, et X représente un atome P ou As, ainsi que, éventuellement, un solvant, et de

b) une phase aqueuse comprenant du peroxyde d'hydrogène.

**2.** Procédé conforme à la revendication 1, dans lequel la dioléfine est choisie parmi le 1,5-hexadiène, le 1,7-octadiène, le 1,9-décadiène, l'éther diallylique, le carbonate de diallyle, les divinylbenzènes, le 1,4-cyclohexadiène et le 1,5-cyclooctadiène.

**3.** Procédé conforme à l'une des revendications 1 et 2, dans lequel, dans la formule (II), Q représente un cation de type ammonium quaternaire comportant des groupes hydrocarbonés qui contiennent au total de 25 à 40 atomes de carbone, et X représente P.

**4.** Procédé conforme à l'une des revendications 1 à 3, dans lequel on utilise un catalyseur de formule (II) dans lequel Q est composé, pour 75 %, de diméthyl-dioctadécyl-ammonium et, pour 25 %, de diméthyl-dihexadécyl-ammonium, et X représente P.

**5.** Procédé conforme à l'une des revendications 1 à 4, dans lequel le rapport molaire du catalyseur de formule (II) au peroxyde d'hydrogène vaut de 1/10 à 1/2500, et en particulier, de 1/200 à 1/800.

**6.** Procédé conforme à l'une des revendications 1 à 5, dans lequel le rapport molaire du peroxyde d'hydrogène à la dioléfine vaut de 0,5/1 à 1,5/1.

**7.** Procédé conforme à l'une des revendications 1 à 6, dans lequel la concentration du peroxyde d'hydrogène dans la phase aqueuse vaut de 0,1 à 70 % en poids.

**8.** Procédé conforme à l'une des revendications 1 à 7, dans lequel la phase organique ne contient aucun solvant ou contient au moins un solvant inerte qui n'est pratiquement pas miscible avec la phase aqueuse, et qui est de préférence choisi parmi les hydrocarbures aromatiques, les hydrocarbures chlorés, les esters alkyliques d'acide carboxylique et les mélanges de tels solvants.

**9.** Procédé conforme à l'une des revendications 1 à 8, dans lequel la concentration de la dioléfine dans la phase organique vaut de 5 à 95 % en poids.

**10.** Procédé conforme à l'une des revendications 1 à 9, qui est mis en oeuvre à une température de 0 °C à 120 °C et/ou sous une pression de 1,013 à 101,3 bars (1 à 100 atm).